# EUROPEAN PATENT SPECIFICATION

(11) **EP 3 458 613 B1**
(45) Date of publication and mention of the grant of the patent: **14.09.2022**
(21) Application number: 17800142.6
(22) Date of filing: 18.05.2017
(51) Int. Cl.: C12Q 1/68, C12Q 1/6825

(54) **METHOD FOR DETECTION OF A PCR PRODUCT**
VERFAHREN ZUR ERKENNUNG EINES PCR-PRODUKTS
PROCÉDÉ DE DÉTECTION D'UN PRODUIT DE PCR

(30) Priority: 18.05.2016 US 201662337917 P
(43) Date of publication of application: 27.03.2019
(73) Proprietor: Integrated Nano-Technologies, Inc., Henrietta, NY 14467 (US)
(72) Inventor: MURANTE, Richard, S., Rochester NY 14625 (US); TANNOUS, Vera, Penfield NY 14526 (US)
(74) Representative: Hauck Patentanwaltspartnerschaft mbB
(86) International application number: PCT/US2017/033255
(87) International publication number: WO 2017/201239

(56) References cited:
- WO-A1-2007/084077
- WO-A2-2006/112818
- WO-A2-2011/046972
- KR-A- 20120 107 501
- US-A1- 2004 185 470
- US-A1- 2005 196 760
- US-A1- 2008 108 055
- US-A1- 2010 184 028
- US-A1- 2015 176 065
- BROWNIE ET AL.: 'The elimination of primer-dimer accumulation in PCR' NUCLEIC ACIDS RESEARCH vol. 25, no. 16, 1997, pages 3235 - 3241, XP055296897
- RIVAS G A ET AL: "ELECTROCHEMICAL BIOSENSORS FOR SEQUENCE-SPECIFIC DNA DETECTION", ANALYTICAL LETTERS, TAYLOR & FRANCIS INC, US, vol. 38, no. 15, 1 January 2005 (2005-01-01), pages 2653-2703, XP009075475, ISSN: 0003-2719, DOI: 10.1080/00032710500371121

## Description

The subject matter disclosed herein relates to a polymerase chain reaction (PCR) process and, more particularly, to a method for nested detection of a PCR product.

PCR is a technique that allows for replicating and amplifying trace amounts of DNA fragments into quantities that are sufficient for analysis. As such, PCR can be used in a variety of applications, such as DNA sequencing and detecting DNA fragment in samples, such as for detection of pathogens in samples.

In operation, PCR involves the use of a series of repeated temperature changes or cycles that cause the DNA to melt or denature, yielding two single-stranded DNA molecules that then act as templates. Primers, short DNA fragments, containing sequences complementary to a target region of DNA along with a DNA polymerase, are used to selectively repeat amplification for a particular DNA region or sequence. Typically, two primers are included in a reaction mixture. The primers are single- stranded sequences, but are shorter than the length of the target region of DNA. The primers bind to a complementary part of the DNA strand and the DNA polymerase binds to the primer-DNA hybrid and begins DNA formation of a new DNA strand complementary to the DNA template strand. The process is repeated until multiple copies of the DNA strands have been created.

However, in some instances, the primers can be subject to hetero-dimerization, in which sequences of the primer bind to each other, rather than to the DNA, resulting in short chains of dimers or artifact amplification products, known as primer dimers. These artifact products can form in the early stages of PCR and subsequently be amplified.
A Method of suppressing artifact amplification products in the PCR phase by the use of PNA clamping probe is taught in US 2015/0176065.
A method which forgoes PCR altogether is taught in WO 2007/084077. Here the original target DNA molecules are tethered to a substrate in a probe, making amplification unnecessary.
Different modified methods of PCR are disclosed in US 2005/0196760 A1 and US 2008/0108055 Al.
The fundamentals of DNA-based electrochemical biosensors are discussed in Rias G A et al: "Electrochemical Biosensors for sequence-specific DNA detection". Analytical Letters, Taylor & Francis INC, US, vol. 38, no. 15, 1 January 2005 (2005-01-01).

An electronic sensor for detection of specific target nucleic acid molecules can include capture probes immobilized on a sensor surface between a set of paired electrodes. An example of a system and method for detecting target nucleic acid molecules is described in U.S. Patent No. 7,645,574. Following PCR, amplified products or amplicons derived from targeted pathogen sequences are captured by the probes via a 5' single-stranded tail, which was incorporated in the molecules during the amplification process by the use of primers made with an internal replication block. Nano-gold clusters, functionalized with a second capture oligonucleotide having a complementary sequence to a universal 5'-tail tagged onto the other end of the amplified product, are used for localized hybridization to only sensor sites having captured amplification products. Subsequently, using a short treatment with a gold developer reagent, the nano-gold clusters serve as catalytic nucleation sites for metallization, which cascades into the development of a fully conductive film. The presence of the gold film shorts the gap between the electrodes and is measured by a drop in resistance, allowing the presence of the captured amplification products can then be measured. However, primer-only artifact products or possible amplicons derived from spurious nucleic acid molecules, with both primers having the requisite 5' tails, can react with such sensors in the same way a DNA target would and can result in false positive results.

### SUMMARY

A method for detecting a nucleic acid molecule in a biological sample includes amplifying a nucleic acid molecule to generate an amplicon having a single 5'-tail and hybridizing the 5'-tail to one of a plurality of capture probes on a surface of a sensor. The amplicon is converted to a single strand molecule and a target-specific catalyst cluster is bound to the single strand molecule. The catalyst cluster is subjected to metallization in order to detect a target nucleic acid.

In an embodiment, a method for detecting a target nucleic acid molecule in a sample with a sensor is disclosed. The sensor includes a first electrode and a second electrode coupled to a sensor surface in a spaced apart arrangement and a plurality of capture probes coupled to the sensor surface between the first electrode and the second electrode. The method includes performing nucleic acid molecule amplification via polymerase chain reaction (PCR) using a first primer having a 5'-tail and a second primer having no 5'-tail to form a plurality of double-stranded amplicons having a first strand with a 5'-tail and a second strand with no tail and hybridizing the plurality of amplicons to the plurality of capture probes. The method further includes converting the plurality of amplicons to a plurality of single strand molecules and binding a catalyst cluster to an interior section of each of the plurality of single strand molecules. In addition, the method includes contacting the plurality of single strand molecules having a catalyst cluster bound thereon with a metal or metal alloy to deposit the metal or metal alloy on the catalyst cluster and determining if an electrical current can be carried between the electrodes. The electrical current between the electrodes indicates the presence of the target nucleic acid molecule in the sample.

In another embodiment, a method for preparing a nucleic acid molecule detector is disclosed. The nucleic acid molecule detector includes a first electrode and a second electrode coupled to a sensor surface in a spaced apart arrangement and a plurality of capture probes coupled to the sensor surface between the first electrode and the second electrode. The method includes receiving a biological sample, amplifying a nucleic acid molecule within the biological sample to generate an amplicon having a single 5'-tail, and binding the 5'-tail of the amplicon to one of the plurality of capture probes. The method additionally includes employing an exonuclease to digest one strand of the amplicon to convert the amplicon to a single strand molecule and synthesizing a target-specific catalyst cluster. The method further includes contacting the catalyst cluster with the single strand molecule of the amplicon to bind the target-specific catalyst cluster to an interior region of the single strand molecule.

An advantage that may be realized in the practice of some disclosed embodiments is reduction or elimination of false positives due to the formation of primer-dimer artifacts or other unintended amplification products.

The above embodiments are exemplary only. Other embodiments are within the scope of the disclosed subject matter.

### BRIEF DESCRIPTION OF THE DRAWINGS

So that the manner in which the features of the invention as defined by the appended claims can be understood, a detailed description of the invention may be had by reference to certain embodiment, some of which are illustrated in the accompanying drawings. It is to be noted, however, that the drawings illustrate only certain embodiments of this invention and are therefore not to be considered limiting of its scope, for the scope of the disclosed subject matter encompasses other embodiments as well. The drawings are not necessarily to scale, emphasis generally being placed upon illustrating the features of certain embodiments of the invention. In the drawings, like numerals are used to indicate like parts throughout the various views.
FIG. 1 is an illustration of an embodiment of a nucleic acid molecule sensor surface;
FIG. 2 is a flowchart illustrating a method of detecting nucleic acid molecules;
FIG. 3 is an illustration of an embodiment of an amplicon having a single 5' tail;
FIG. 4 is an illustration of the sensor surface of FIG. 1 having the amplicon of FIG. 3 coupled thereto;
FIG. 5 is an illustration of the sensor surface of FIG. 4 with the amplicon converted to a single strand molecule;
FIG. 6 is an illustration of the sensor surface of FIG. 5 with a catalyst cluster coupled to the single strand molecule;
FIG. 7A is an illustration of an embodiment of a method of forming a catalyst cluster;
FIG. 7B is an illustration of an embodiment of another method of forming a catalyst cluster;
FIG. 8A is an illustration of an embodiment of a method of forming a multiplexed catalyst cluster;
FIG. 8B is an illustration of an embodiment of a method of mixing multiplexed catalyst clusters;
FIG. 8C is an illustration of an embodiment of a method of multiple site nesting of multiplexed catalyst clusters on single strand amplicon molecules;
FIG. 9 is an illustration of a portion of a ribosomal gene used for testing;
FIG. 10 is a photograph of microchip surfaces resulting from testing the effects of time on exonuclease digestion of amplicon strands;
FIG. 11 is a photograph of microchip surfaces resulting from testing the effects of varying exonuclease concentration;
FIG. 12 is a photograph of microchip surfaces comparing the results of two different catalyst cluster reagents;
FIG. 13A is a photograph of a gel analysis of replicates of RT-PCR for dengue viral RNA in multiplexed reaction using pan-flavivirus primers mixed with Cal/Bun primers; and
FIG. 13B is a photograph of a gel analysis of replicates of RT-PCR for LaCrosse RNA in multiplexed reaction using pan-flavivirus primers mixed with Cal/Bun virus primers.

### DETAILED DESCRIPTION

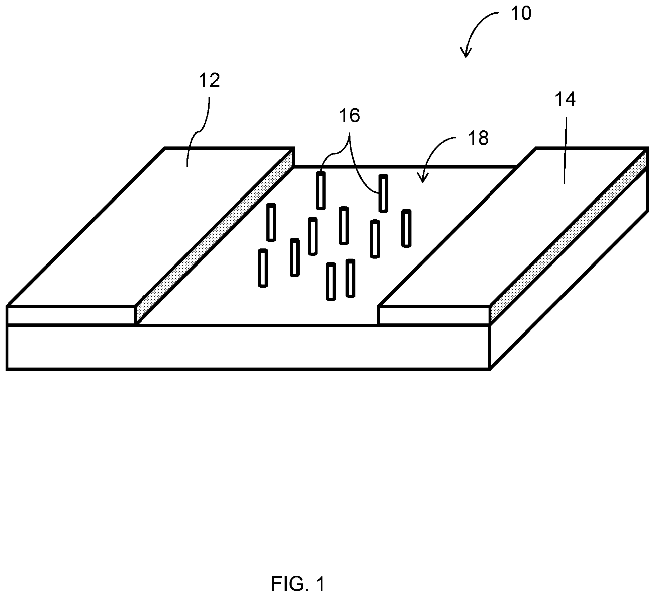

FIG. 1 illustrates an embodiment of a detector sensor microchip 10. In this embodiment, the microchip 10 includes a first electrode 12 and a second electrode 14 positioned so that the first 12 and second 14 electrode do not contact each other, with a plurality of capture probes 16 in the form of a functionalized oxide surface allowing attachment and immobilization of capture probe molecules 16 on the sensor surface 18 between the first electrode 12 and the second electrode 14. The capture probes 16 are designed to capture PCR amplified products via interaction with 5' tails incorporated during the amplification process.

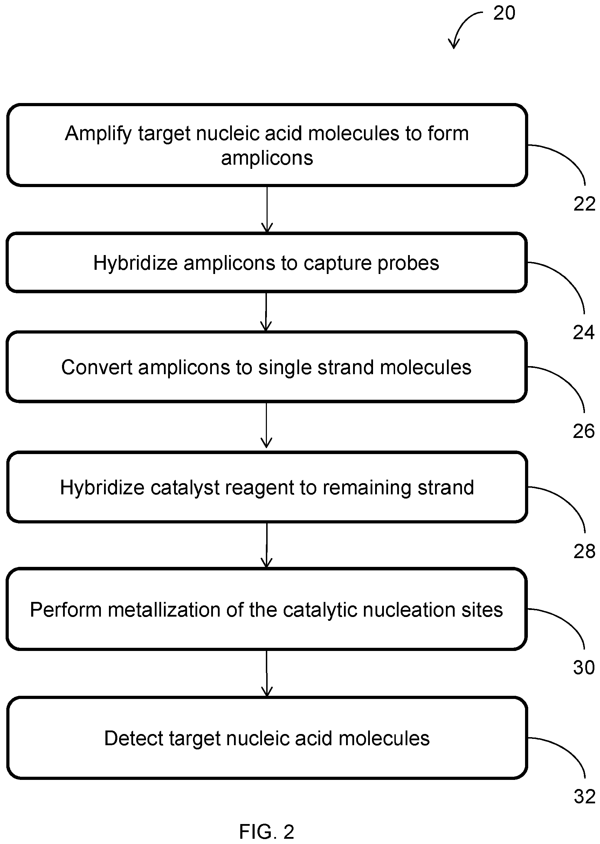

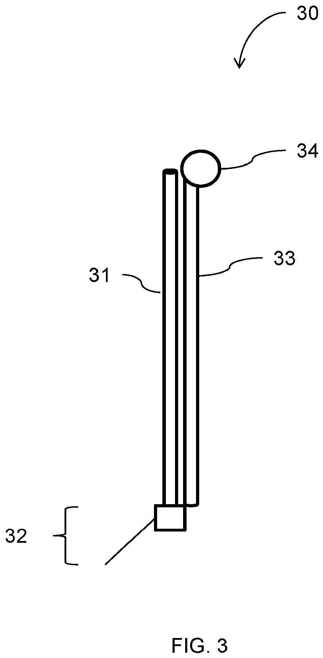

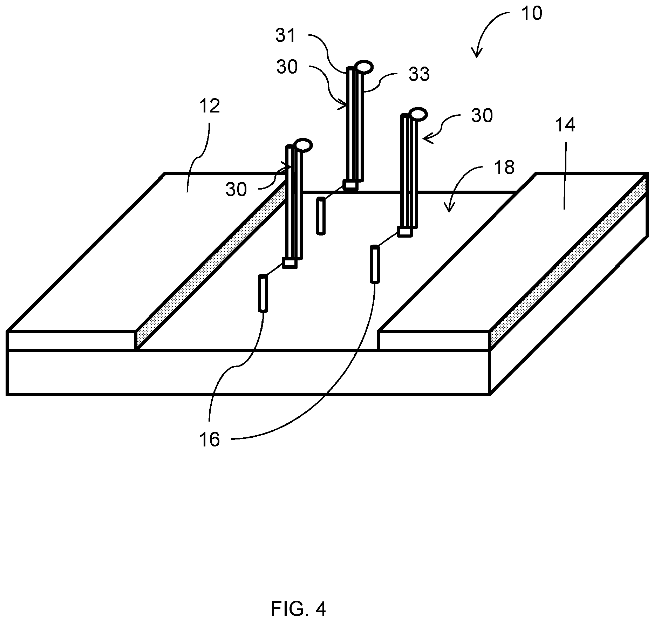

FIG. 2 illustrates an embodiment of a method 20 for detection of target nucleic acid molecules. At block 22, target nucleic acid molecules collected from a biological sample are amplified via PCR. The biological sample could be any suitable type of material, such as blood, mucous, and skin, among others. It is to be understood that any suitable type of PCR methodology can be employed. In this embodiment, two primers are used during the PCR process. The first primer is synthesized as a 5'-tailed oligonucleotide with an internal replication block and the second primer is a non-tailed oligonucleotide. In an example, the second primer has a 5' phosphate group. Multiple cycles are performed until a plurality of double-stranded amplicons 30 (replications of the target nucleic acid molecules) are formed having a first strand 31 with a 5' tail 32 and a second strand 33 extended from the non-tailed primer, as illustrated in FIG. 3. In an example, illustrated in FIG. 3, the second strand 33 has a 5'-phosphate group 34. In another example, not illustrated, the second strand 33 does not have a 5'-phosphate group. Returning to FIG. 2, at block 24, the amplicons 30 are hybridized to the capture probes 16 on the surface of the detector microchip 10, illustrated in FIG. 1. In particular, the 5' tails of the amplicons 30 bind to the capture probes 16, as illustrated in FIG. 4.

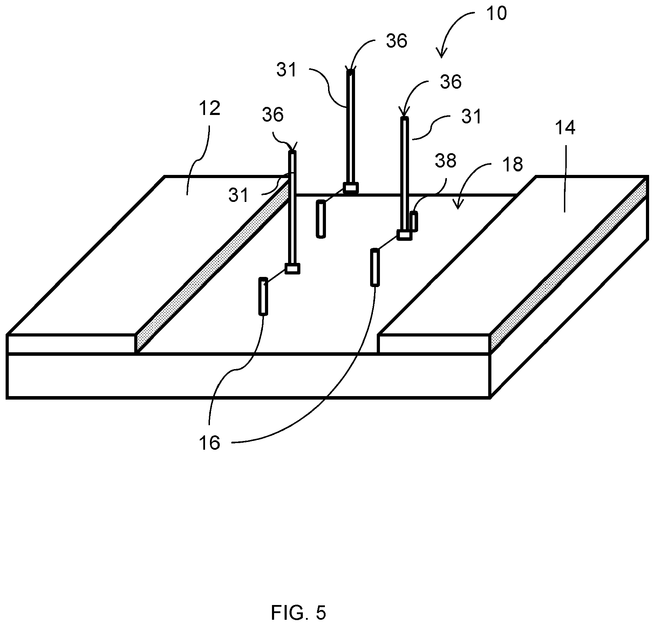

At block 26, the hybridized amplicons 30 are converted to single strand molecules 36, as illustrated in FIG. 5. In an example, rather than employing heat, which would denature the amplicons 30 off of the capture probes 16, a 5'-to-3' directional helicase is used to convert the amplicons 30 to single strand molecules 36. In another example, an exonuclease is employed to convert the amplicons 30 to single strand molecules 36. In this example, the exonuclease has 5' to 3' directionality and preferentially digests the strand 33 extended from the non-tail primer. Because the tailed strand 31 is bound to the capture probe 16, the exonuclease cannot access the 5' end of the strand 31, and therefore cannot digest the strand 31, preventing degradation of the tailed strand 31. In an example, depending on nuclease processivity or steric hindrance at a particular distance from the sensor surface 18, the strand 33 may experience incomplete digestion, resulting in a remaining portion 38 of the digested strand 33. In an example, the strand 33 extended from the non-tail primer is synthesized with a 5' phosphate and the exonuclease is a lambda exonuclease.

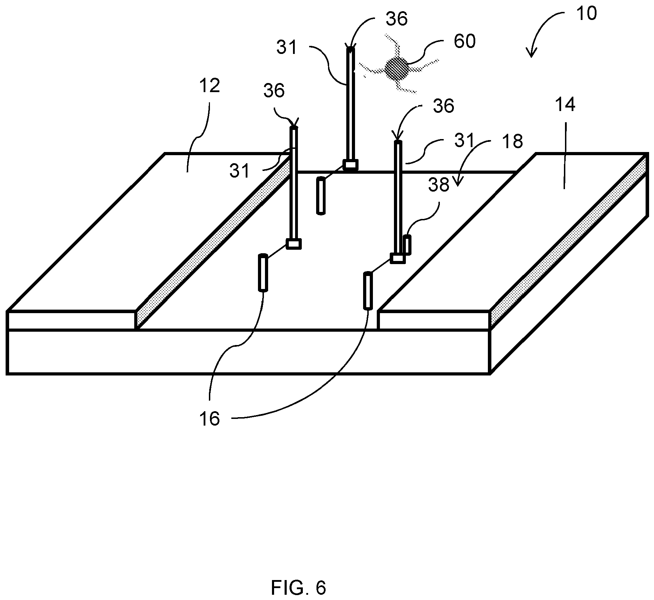

Digestion of the strand 33 exposes the internal sequence region of the tailed strand 31. At block 28 of the method 20 (FIG. 2), a catalyst reagent, such as a gold catalyst reagent, is directly hybridized to the tailed strand 31, as illustrated in FIG. 6. In an embodiment, the catalyst reagent is in the form of catalyst clusters. In an embodiment, a single catalyst cluster 60 binds to the tailed strand 31. In another embodiment, and depending on the length of the tailed strand 31, a plurality of catalyst clusters 60 bind to each tailed strand 31.

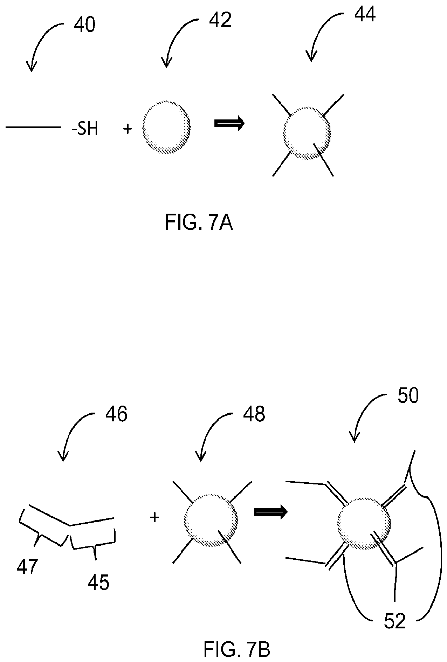

Since a generic oligonucleotide is not suitable for binding to internal sequences within the amplicons, the catalyst clusters are target-specific, i.e., the catalyst clusters bind to specific target sequences in the strand 31. Because the primer-dimer artifacts do not include these target sequences, the catalyst clusters 60 do not bind to primer-dimer artifacts, thus avoiding potential false positive measurements. As illustrated in FIG. 7A, in one example, a thiol-modified oligonucleotide 40 can be reacted with a catalytic cluster 42 to form a catalyst cluster 44 functionalized with at least 20 oligonucleotides. In another example, illustrated in FIG. 7B, a universal oligonucleotide 46 that possesses a universal cluster binding sequence 45 and amplicon-specific bind sequence 47 can be hybridized with a base generic cluster 48 to form a catalyst cluster 50. As a result, each new oligonucleotide is concatenated at the 3'-end with an adaptor specific sequence complementary to the cluster generic oligonucleotide. While the resulting catalyst cluster 50 in FIG. 7B is depicted as having four hybridized adaptor oligonucleotides 52, it is to be understood that the actual number of adaptors will depend on cluster size, which limits the number of capture oligonucleotides on the base cluster, and the stoichiometry of adaptor oligonucleotides added to the cluster.

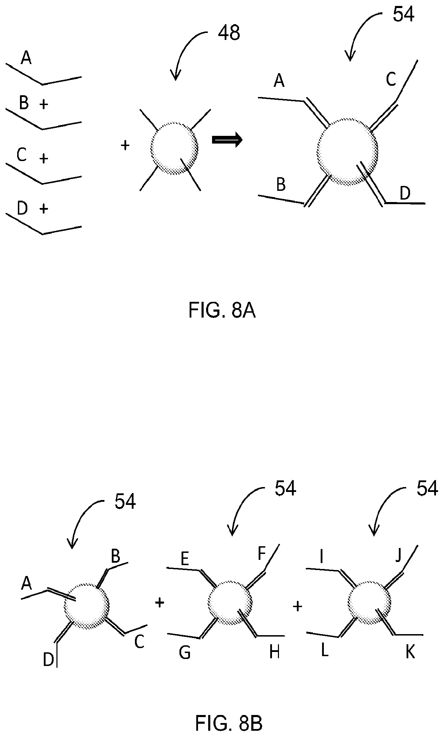

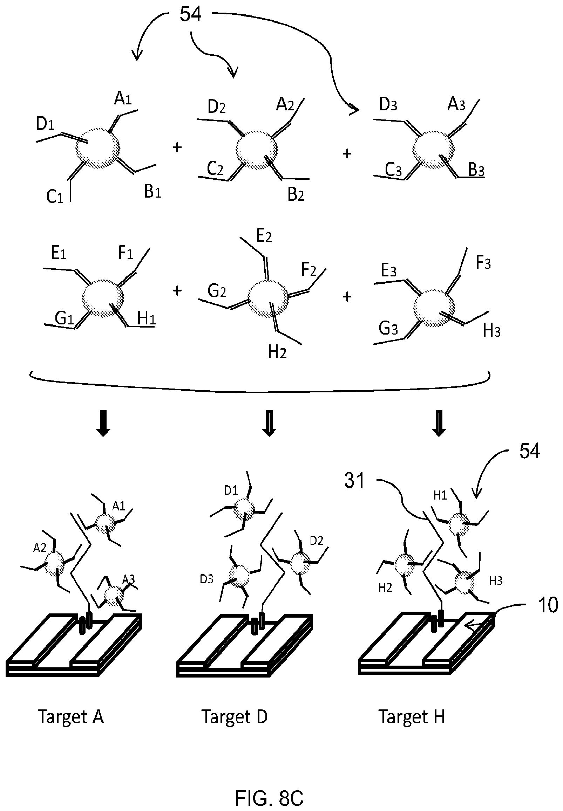

As illustrated in FIG. 8A, the base generic clusters 48 can be prepared with mixtures of probe oligonucleotides A, B, C, D to form a catalyst cluster 54 with multiplexing capabilities. Depending on cluster size, which determines the surface area available for functionalization, it is possible to load multiple different probes onto a single cluster. In order to support efficient metallization reactions, the ratio for the number of each probe type per cluster may require optimization. As illustrated in FIG. 8B, tailored mixtures of multiplexed clusters 54 can be created. The mixtures of multiplexed clusters 54 can both multiplex for a large number of targeted amplicons and populate each targeted amplicon with multiple clusters, as illustrated in FIG. 8C.

Returning to FIG. 2, at block 30, metallization of the catalyst clusters 60, which serve as catalytic nucleation sites, can be performed to form a conductive film and resistance between the electrodes 12, 14 (FIG. 1) can be measured to detect target nucleic acid molecules at block 32. In an example, the catalyst clusters 60 are gold clusters and a gold developer reagent is applied to the catalyst clusters 60 to cascade into the development of the conductive film, which in this example is a gold film. In this example, the presence of the gold film electrically shorts the gap between the electrodes 12, 14 and is measured by a drop in resistance. In this example, a negative sensor has a resistance of more than one million ohms and a positive sensor has a resistance of about one thousand ohms.

### EXAMPLE:

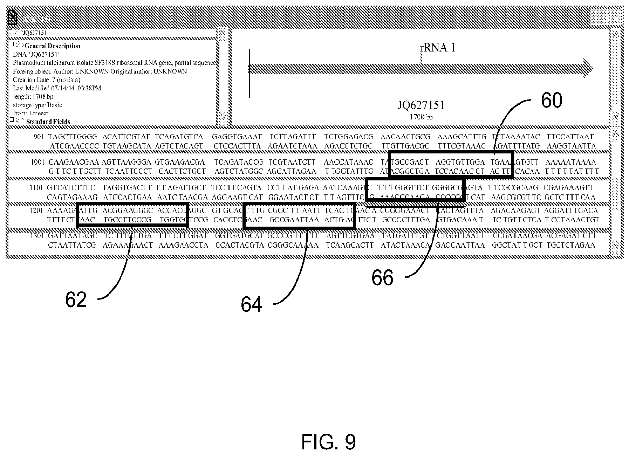

An existing test developed for plasmodium faliciprium was used to test the method 20 described above. FIG. 9 is a screenshot taken from the INVITROGEN^{™} program showing a portion of the 18S ribosomal gene to which primers (shown in bold lettering) are designed. Typically, the two primers, forward primer 60 and reverse primer 62, used in PCR for this method each have a sensor and catalyst binding 5'-tail. However, using the method 20 described above, a new reverse primer 64 located downstream of the original reverse primer 62 was identified. Synthesis of the new reverse primer 64 omitted the 5' tail for catalyst binding while including a 5'-phosphorl group to facilitate exonuclease degradation.

Because the 5'-tail on the original reverse primer 62 hybridizes to a universal catalyst reagent, modification of the catalyst cluster for use with the method 20 was accomplished by pre-hybridization of the original reverse primer oligonucleotide onto a universal cluster to form target-specific catalyst gold clusters. Preparation of these target-specific catalyst gold clusters included a heated incubation with 1000 fold molar excess of the primer 62, cooling to room temperature, and removal of unbound excess oligonucleotide by washing, repeated twice, via high-speed centrifugation and resuspension with the final reagent buffer. Similarly, a second catalyst reagent 66 with specificity towards a different sequence element located upstream of the first cluster binding sequence was prepared. The ability of this second cluster to effect metallization and detection served as only a preliminary attempt to assess the processivity of the exonuclease in the digestion of sensor-bound amplicons. With this particular derived amplicon, the nuclease must have digested to within at least 95 nucleotides of completely degrading the extraneous DNA strand, leaving a single-stranded tract of about eighty nucleotides available for cluster binding.

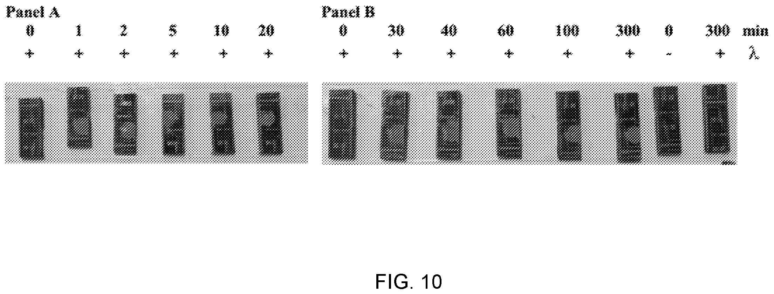

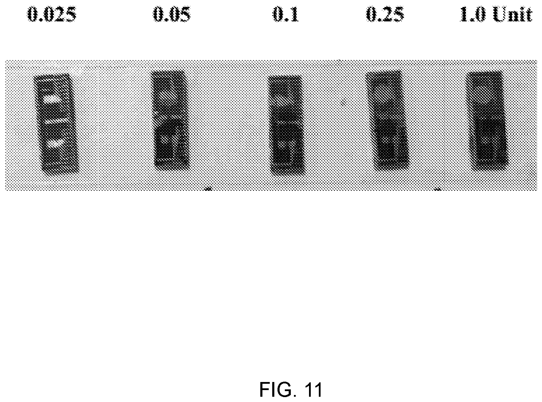

Select 5' to 3' exonucleases with suitable properties to perform a digestion as per the method 20 were assessed. Two suitable commercially available exonucleases, Lambda and T7, were identified. Lambda was used in this example. FIGs. 10 and 11 illustrate the results of this experiment.

FIG. 10 illustrates the results of time coarse experiments of Lambda exonuclease treatments on derived amplicons hybridized to microchips. All microchips in Panel A and Panel B were hybridized for five minutes at 45 degrees Celsius with 100 ng of the amplicon, washed twice by dipping into a 10 mL volume of hybridization buffer, and dried under a nitrogen gas stream. In a humidified petri dish held in a 37 degree Celsius incubator, the microchips were spotted with 25 µL Lambda reaction solution containing one unit of the exonuclease. One unit of exonuclease is defined as the amount of enzyme required to produce 10 nmol of acid-soluble deoxyribonucleotide from a double-stranded substrate in a total reaction volume of 50 µL in 30 minutes at 37 degrees Celsius in IX lambda Exonuclease Reaction Buffer with 1 µg sonicated duplex [³H]-DNA. After incubation for a designated time, indicated above each microchip, the reactions were quenched by transferring the microchips into a petri dish with 20 mL of hybridization buffer. Subsequently, for metallization, catalyst hybridization with clusters modified with the primer oligonucleotide was performed for five minutes at 45 degrees Celsius, two washes were performed by swirling the chips in petri dishes containing hybridization buffer, and then gold development was performed for four minutes at 55 degrees Celsius with 25 µL of developer reagent placed on the chip surface. FIG. 11 illustrates the effect of titration of the Lambda exonuclease on the gold development of target amplicons that were hybridized on the surface of the microchips. The microchips were hybridized as described above with regard to FIG. 10. However, the units of Lambda exonuclease used on each microchip illustrated in FIG. 11 was varied as indicated above each microchip.

One finding of these experiments was that the Lambda exonuclease could not digest the 5'-tail primer extended strand, which would have caused a loss in the capacity to capture the catalyst reagent. This finding is supported by the longer time coarse digestion experiment illustrated in FIG. 10, which shows there is no loss of chip metallization, even with the most extensive Lambda incubation time of 300 minutes.

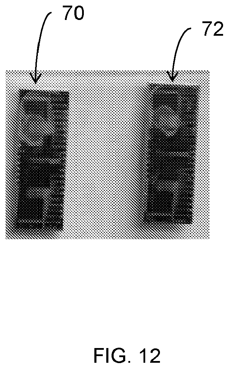

The ability of the adaptor modified clusters to hybridize to a more internal site within the 5'-tailed strand were investigated by preparation of the second cluster reagent, described above. This second cluster was formed to bind about thirty nucleotides proximal of the hybridization site of the first cluster. The results of this investigation of illustrated in FIG. 12, which compares development of a first microchip 70 processed with the first cluster reagent and a second microchip 72 processed with the second cluster reagent. Comparison of the first microchip 70 and second microchip 72 shows that both clusters performed equally well yielding comparable gold spots on the respective treated microchips. In addition, the results established that, on a majority of the hybridized amplicons, the Lambda exonuclease digested a minimum of ninety bases and was not inhibited while approaching the microchip surface. In addition, the longer stretch of single-stranded DNA with which the second cluster interacted did not appear to affect bind of the second cluster relative to binding of the first cluster.

Using the method described above for *Plasmodium falciparum* (*P.f.)*, fifty test cartridge runs were performed. Negative and positive samples consisted respectively of either 10 µL of water or a 1 µL blood culture of *P.f.* (10⁵) cells diluted in a buffer to 10 µL. After pipetting and sealing a sample into the test cartridge, a fully automated assay, including sample preparation, PCR amplification, and microchip hybridization, nuclease digestion, and metallization reactions, was carried out. Post assay electrical measurements were performed by removal of each microchip board from the cartridge after each test run and visually inspecting each microchip before placing the microchip on a probe station for collection of electrical results. Table 1 presents the raw data from the fifty assays. As indicated by "NO TEST" in the "results" column of Table 1, certain assays were excluded due to machine failure or operator mistakes, as further indicated in the "Notes" column in Table 1. Table 2 indicates the percentages of correct true positives and negatives obtained. As illustrated in Table 2, using a modified, single-tailed amplicons resulted in 100% correct negative measurements and 88% correct positive measurements.

**TABLE 1:**

| E-test # of shorted sensors | | | | | | |
|---|---|---|---|---|---|---|
| Date | TEST | TARGET | CTL | NCOMP | RESULT | Notes |
| 3/14/16 | POS | 10 | 0 | 0 | CORRECT | |
| 3/14/16 | POS | 10 | 0 | 0 | CORRECT | |
| 3/14/16 | NEG | 0 | 0 | 0 | CORRECT | |
| 3/14/16 | NEG | 0 | 0 | 0 | CORRECT | |
| 3/14/16 | NEG | 0 | 0 | 0 | CORRECT | |
| 3/15/16 | POS | 0 | 0 | 0 | INCORRECT | No sign of target development - Benchtop development indicates SP-PCR ok |
| 3/15/16 | POS | 0 | 0 | 0 | NO TEST | Cartridge leaked. Benchtop development of hyb buffer indicates SP-PCR ok |
| 3/15/16 | NEG | | | | NO TEST | PCR Temp Timeout |
| 3/15/16 | NEG | 0 | 0 | 0 | CORRECT | |
| 3/15/16 | NEG | 0 | 0 | 0 | CORRECT | |
| 3/15/16 | NEG | 0 | 0 | 0 | CORRECT | |
| 3/16/16 | POS | 10 | 0 | 0 | CORRECT | |
| 3/16/16 | POS | 10 | 0 | 0 | CORRECT | |
| 3/16/16 | NEG | 0 | 0 | 0 | CORRECT | |
| 3/17/16 | POS | 10 | 0 | 0 | CORRECT | |
| 3/17/16 | POS | 10 | 0 | 0 | CORRECT | |
| 3/17/16 | NEG | 0 | 0 | 0 | CORRECT | |
| 3/17/16 | NEG | 0 | 0 | 0 | CORRECT | |
| 3/18/16 | POS | 10 | 0 | 0 | CORRECT | |
| 3/18/16 | POS | 10 | 0 | 0 | CORRECT | |
| 3/18/16 | NEG | 0 | 0 | 0 | CORRECT | |
| 3/18/16 | NEG | 0 | 0 | 0 | CORRECT | |
| 3/18/16 | NEG | 0 | 0 | 0 | CORRECT | |
| 3/21/16 | POS | 10 | 0 | 0 | CORRECT | |
| 3/21/16 | POS | 10 | 0 | 0 | CORRECT | |
| 3/21/16 | NEG | 0 | 0 | 0 | NO TEST | Developer misloaded - turned out ok when completed on benchtop |
| 3/21/16 | NEG | 0 | 0 | 0 | CORRECT | |
| 3/21/16 | NEG | 0 | 0 | 0 | CORRECT | |
| 3/22/16 | POS | 10 | 0 | 0 | CORRECT | |
| 3/22/16 | POS | 10 | 0 | 0 | CORRECT | |
| 3/22/16 | NEG | 0 | 0 | 0 | CORRECT | |
| 3/22/16 | NEG | 0 | 0 | 0 | CORRECT | |
| 3/22/16 | NEG | 0 | 0 | 0 | CORRECT | |
| 3/23/16 | POS | 9 | 0 | 0 | CORRECT | |
| 3/23/16 | POS | 0 | 0 | 0 | NO TEST | Weak development - machine ran unusually slow (SD card issues) |
| 3/23/16 | POS | 10 | 0 | 0 | CORRECT | |
| 3/23/16 | NEG | 0 | 0 | 0 | CORRECT | |
| 3/23/16 | NEG | 0 | 0 | 0 | CORRECT | |
| 3/23/16 | POS | 10 | 0 | 0 | CORRECT | |
| 3/23/16 | POS | 10 | 0 | 0 | CORRECT | |
| 3/23/16 | POS | 10 | 0 | 0 | CORRECT | |
| 3/28/16 | POS | 0 | 0 | 0 | INCORRECT | No development |
| 3/28/16 | POS | 0 | 0 | 0 | INCORRECT | No development |
| 3/28/16 | POS | | | | NO TEST | Motor COM error, Motortimeout |
| 3/28/16 | POS | 10 | 0 | 0 | CORRECT | |
| 3/28/16 | NEG | | | | NO TEST | Syringe timeout error |
| 3/31/16 | POS | 10 | 0 | 0 | CORRECT | |
| 3/31/16 | POS | 10 | 0 | 0 | CORRECT | |
| 3/31/16 | POS | 6 | 0 | 0 | CORRECT | Light development |
| 3/31/16 | POS | 10 | 0 | 0 | CORRECT | |

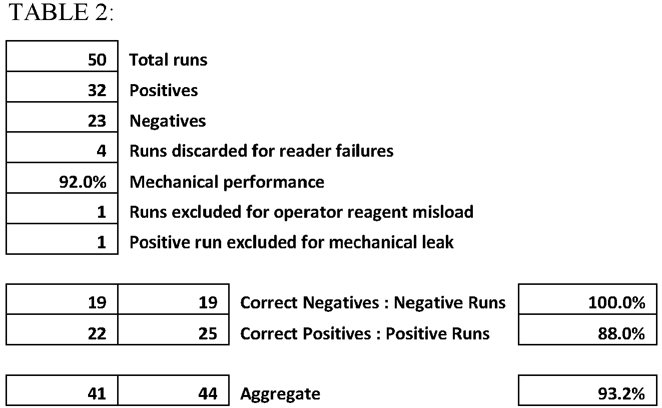

### EXAMPLE 2:

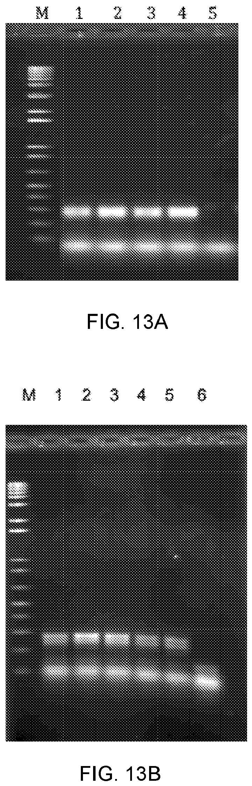

Various combinations of multiplexed reverse transcription PCR (RT-PCR) were assessed using primer sets designed for development of a pan-flavivirus/pan-alphavirus/pan-bunyavirus test. A total of nine primers were used in this assay, with all assayed materials derived from the homogenization, using ultrasonically driven bead-beating, of pooled six to eight mosquitoes that were spiked or non spiked with a virus, VEE-TC83, Dengue, or LaCrosse Virus. Nucleic acid material was isolated from the homogenates using magnetic particle purification, desalted by gel-filtration, and used in the RT-PCR amplification with the appropriate multiplexed primer sets, either pan-flavivirus plus bunyavirus primers or alpha primers, to generate single-stranded 5'-tailed amplicons. The findings indicate that the primer sets for the pan-alpha and pan-flavivirus tests inhibit one another during PCR amplification. To address this problem, the test cartridge provides two separate PCR chambers allowing for interfering primer sets to be run separately and mixed prior to hybridization on the sensor chip. FIG. 13A illustrates gel analysis of gene replicates of RT-PCR for dengue viral RNA (lanes 1-4), purified from spike mosquitoes, in multiplexed reaction using pan-flavivirus primers mixed with the Cal/Bun primers. Lane 5 is a negative control with no RNA input. FIG. 13B illustrates gel analysis of RT-PCR for LaCrosse RNA (lanes 1-5), purified from spiked mosquitoes, in multiplexed reaction using pan-flavivirus primers mixed with the Cal/Bun primers. Lane 6 is a negative control, no RNA input. The findings indicate that combinations of pan-bunyavirus and pan-flavivirus primers are compatible in PCR reactions.

Possible advantages of the above described method include reduction or elimination of false positive measurements due to primer-dimer artifact formation.

To the extent that the claims recite the phrase "at least one of' in reference to a plurality of elements, this recitation is intended to mean at least one or more of the listed elements, and is not limited to at least one of each element. For example, "at least one of an element A, element B, and element C," is intended to indicate element A alone, or element B alone, or element C alone, or any combination thereof. "At least one of element A, element B, and element C" is not intended to be limited to at least one of an element A, at least one of an element B, and at least one of an element C.

## Claims

1. A method for detecting a target nucleic acid molecule in a sample with a sensor comprising a first electrode and a second electrode coupled to a sensor surface in a spaced apart arrangement and a plurality of capture probes coupled to the sensor surface between the first electrode and the second electrode, the method comprising:
performing nucleic acid molecule amplification via polymerase chain reaction (PCR) using a first primer having a 5'-tail and comprising an internal replication block configured to stop a polymerase action short of the 5'-tail, and a second primer being complimentary to a region of the target nucleic acid molecule and having no 5'-tail, wherein the PCR forms a plurality of double-stranded amplicons with a single-strand 5'-tail, wherein each double stranded amplicon comprises
a first strand including an internal target sequence, wherein the first strand further comprises the 5'-tail, and
a second strand with no tail;
applying the plurality of double-stranded amplicons formed by the PCR to the sensor surface;
hybridizing the plurality of double-stranded amplicons to the plurality of capture probe sequences, wherein the single-strand 5'-tails of the plurality of double-stranded amplicons bind to the plurality of capture probe sequences;
converting the plurality of double-stranded amplicons to a plurality of single strand molecules using a 5'-3' exonuclease with specificity for double-stranded DNA to expose the target sequence of the first strand, wherein the 5'-tails of the plurality of single-strand molecules remain bound to the plurality of capture probe sequences and an opposing 3' end is unbound;
hybridizing a catalyst cluster to the internal target sequence of each of the plurality of single strand molecules;
contacting the plurality of single strand molecules having a catalyst cluster bound thereon with a metal or metal alloy, wherein the metal or metal alloy binds to the catalyst cluster to form a conductive metal film between the first electrode and the second electrode; and
determining if an electrical current can be carried between the electrodes, the electrical current between the electrodes indicating presence of the target nucleic acid molecule in the sample.

2. The method of claim 1, wherein converting the plurality of amplicons to single strand molecules comprises employing an exonuclease to digest the second strand with no tail.

3. The method of claim 1, wherein the catalyst cluster comprises a catalyst gold cluster and wherein the metal comprises gold.

4. The method of claim 1, further comprising forming a target specific catalyst cluster configured to bind to an interior region of the first strand of the amplicon.

5. The method of claim 1, wherein the catalyst cluster is a generic cluster having an adaptor oligonucleotide coupled thereto.

6. The method of claim 5, wherein the generic cluster has a plurality of oligonucleotides coupled thereto, each oligonucleotide configured to target a different amplicon.

7. The method of claim 1, wherein binding the catalyst cluster comprises binding a plurality of catalyst clusters to interior sections of each of the single strand molecules.

## Patentansprüche

1. Verfahren zur Erkennung eines Zielnukleinsäuremoleküls in einer Probe mit einem Sensor, der eine erste Elektrode und eine zweite Elektrode, die in einer voneinander beabstandeten Anordnung an eine Sensoroberfläche gekoppelt sind, und eine Vielzahl von Fangsonden umfasst, die an die Sensoroberfläche zwischen der ersten Elektrode und der zweiten Elektrode gekoppelt sind, wobei das Verfahren umfasst:
Durchführen von Nukleinsäuremolekülamplifikation über Polymerasekettenreaktion (PCR) unter Verwendung eines ersten Primers, der ein 5'-Ende aufweist und einen internen Replikationsblock umfasst, der dazu ausgestaltet ist, eine Polymeraseaktion kurz vor dem 5'-Ende anzuhalten, und eines zweiten Primers, der komplementär zu einer Region des Zielnukleinsäuremoleküls ist und kein 5'-Ende aufweist, wobei die PCR eine Vielzahl von doppelsträngigen Amplicons mit einem einsträngigen 5'-Ende bildet, wobei jedes doppelsträngige Amplicon umfasst:
einen ersten Strang, der eine interne Zielsequenz umfasst, wobei der erste Strang ferner das 5'-Ende umfasst, und
einen zweiten Strang ohne Ende;
Aufbringen der Vielzahl von durch die PCR gebildeten doppelsträngigen Amplicons auf die Sensoroberfläche;
Hybridisieren der Vielzahl von doppelsträngigen Amplicons an die Vielzahl von Fangsondensequenzen, wobei die einsträngigen 5'-Enden der Vielzahl von doppelsträngigen Amplicons sich an die Vielzahl von Fangsondensequenzen binden;
Umwandeln der Vielzahl von doppelsträngigen Amplicons in eine Vielzahl von einsträngigen Molekülen unter Verwendung einer 5'-3'-Exonuklease mit Spezifizität für doppelsträngige DNA, sodass die Zielsequenz des ersten Stranges freigelegt wird, wobei die 5'-Enden der Vielzahl von einsträngigen Moleküle an die Vielzahl von Fangsondensequenzen gebunden bleiben und ein entgegengesetztes 3'-Ende entbunden wird;
Hybridisieren eines Katalysator-Clusters an die interne Zielsequenz von jeder der Vielzahl von einsträngigen Molekülen;
Inkontaktbringen der Vielzahl von einsträngigen Molekülen, die einen daran gebundenen Katalysator-Cluster aufweisen, mit einem Metall oder einer Metalllegierung, wobei das Metall oder die Metalllegierung sich so an den Katalysator-Cluster bindet, dass ein leitfähiger Metallfilm zwischen der ersten Elektrode und der zweiten Elektrode gebildet wird; und
Bestimmen, ob ein elektrischer Strom zwischen den Elektroden getragen werden kann, wobei der elektrische Strom zwischen den Elektroden das Vorhandensein des Zielnukleinsäuremoleküls in der Probe anzeigt.

2. Verfahren nach Anspruch 1, wobei das Umwandeln der Vielzahl von Amplicons in einsträngige Moleküle das Einsetzen einer Exonuklease für den Verdau des zweiten Stranges ohne Ende umfasst.

3. Verfahren nach Anspruch 1, wobei der Katalysator-Cluster einen Katalysatorgold-Cluster umfasst und wobei das Metall Gold umfasst.

4. Verfahren nach Anspruch 1, das ferner das Bilden eines zielspezifischen Katalysator-Clusters umfasst, der so ausgestaltet ist, dass er sich an eine interne Region des ersten Stranges des Amplicons bindet.

5. Verfahren nach Anspruch 1, wobei der Katalysator-Cluster ein generischer Cluster ist, der ein daran gekoppeltes Adaptor-Oligonukleotid aufweist.

6. Verfahren nach Anspruch 5, wobei der generische Cluster eine Vielzahl von daran gekoppelten Oligonukleotiden aufweist, wobei jedes Oligonukleotid so ausgestaltet ist, dass es auf ein unterschiedliches Amplicon abzielt.

7. Verfahren nach Anspruch 1, wobei das Binden des Katalysator-Clusters das Binden einer Vielzahl von Katalysator-Clustern an interne Abschnitte von jedem der einsträngigen Moleküle umfasst.

## Revendications

1. Procédé de détection d'une molécule d'acide nucléique cible dans un échantillon à l'aide d'un capteur comprenant une première électrode et une deuxième électrode accouplées à une surface de capteur dans un agencement espacé et une pluralité de sondes de capture accouplées à la surface de capteur entre la première électrode et la deuxième électrode, le procédé comprenant :
l'exécution d'une amplification de molécule d'acide nucléique via une réaction en chaîne par polymérase (PCR) à l'aide d'un premier apprêt présentant une queue 5' et comprenant un bloc de réplication interne configuré pour arrêter une action par polymérase sans la queue 5', et un deuxième apprêt étant complémentaire à une région de la molécule d'acide nucléique cible et ne présentant pas de queue 5', où lequel le PCR forme une pluralité d'amplicons à deux brins avec une queue 5' à brin unique, où chaque amplicon à deux brins comprend
un premier brin incluant une séquence cible interne, où le premier brin comprend en outre la queue 5', et
un deuxième brin sans queue ;
l'application de la pluralité d'amplicons à deux brins formés par le PCR à la surface de capteur ;
l'hybridation de la pluralité d'amplicons à deux brins à la pluralité de séquences de sondes de capture, où les queues 5' à brin unique de la pluralité d'amplicons à deux brins se lient à la pluralité de séquences de sondes de capture ;
la conversion de la pluralité d'amplicons à deux brins en une pluralité de molécules de brin unique à l'aide d'une exonucléase 5'-3' avec une spécificité pour un ADN à deux brins pour exposer la séquence cible du premier brin, où les queues 5' de la pluralité de molécules à brin unique restent liées à la pluralité de séquences de sondes de capture et une extrémité 3' opposée n'est pas liée ;
l'hybridation d'un agrégat catalytique à la séquence cible interne de chacune de la pluralité de molécules à brin unique ;
le mise en contact de la pluralité de molécules à brin unique présentant un agrégat catalytique lié à celles-ci avec un métal ou un alliage de métaux, où le métal ou l'alliage de métaux se lie à l'agrégat catalytique pour former un film métallique conducteur entre la première électrode et la deuxième électrode ; et
la détermination si un courant électrique peut circuler entre les électrodes, le courant électrique entre les électrodes indiquant la présence de la molécule d'acide nucléique cible dans l'échantillon.

2. Procédé selon la revendication 1, dans lequel la conversion de la pluralité d'amplicons en des molécules à brin unique comprend l'emploi d'une exonucléase pour absorber le deuxième brin sans queue.

3. Procédé selon la revendication 1, dans lequel l'agrégat catalytique comprend un agrégat d'or catalytique et dans lequel le métal comprend de l'or.

4. Procédé selon la revendication 1, comprenant en outre la formation d'un agrégat catalytique spécifique cible, lequel est configuré pour se lier à une région interne du premier brin de l'amplicon.

5. Procédé selon la revendication 1, dans lequel l'agrégat catalytique est un agrégat générique comportant un oligonucléotide adaptateur accouplé à celui-ci.

6. Procédé selon la revendication 5, dans lequel l'agrégat générique comporte une pluralité d'oligonucléotides accouplés à celui-ci, chaque oligonucléotide étant configuré pour cibler un amplicon différent.

7. Procédé selon la revendication 1, dans lequel la liaison de l'agrégat catalytique comprend la liaison d'une pluralité d'agrégats catalytiques à des sections internes de chacune des molécules à brin unique.
